# EUROPEAN PATENT APPLICATION

(11) **EP 4 692 308 A1**
(43) Date of publication of application: **11.02.2026**
(21) Application number: 24779538.8
(22) Date of filing: 14.03.2024
(51) Int. Cl.: C12N 1/16, C12P 1/02, C12P 7/6436

(54) **HIGH OIL-PRODUCING STRAIN BELONGING TO GENUS LIPOMYCES, AND METHOD FOR PRODUCING OIL USING HIGH OIL-PRODUCING STRAIN**

(30) Priority: 29.03.2023 JP 2023053911
(71) Applicant: The Niigata Institute of Science and Technology, Niigata-shi, Niigata 956-8603 (JP); NISSIN FOODS HOLDINGS CO., LTD., Yodogawa-ku Osaka-shi, Osaka 532-8524 (JP)
(72) Inventor: TAKAKU, Hiroaki, Nijigata, 956-8603 (JP); MINE, Kentaro, Osaka, 532-8524 (JP); TAKI, Hiroya, Osaka, 532-8524 (JP)
(74) Representative: Marks & Clerk LLP
(86) International application number: PCT/JP2024/010112
(87) International publication number: WO 2024/203409

(57) **Abstract**

An object of the present invention is to obtain a novel mutant strain of yeast belonging to the genus *Lipomyces,* the novel mutant strain having higher oil productivity than known high oil-producing mutant strains, and to provide a method of producing a palm oil alternative using the novel mutant strain. The novel high oil-producing mutant strain according to the present invention is a mutant strain of yeast belonging to the genus *Lipomyces* and has been deposited at the National Institute of Technology and Evaluation, Patent Microorganisms Depositary, under accession number NITE BP-03796. This novel high oil-producing mutant strain is obtained as follows: CBS1807, which is a wild-type strain of *Lipomyces starkeyi,* is irradiated with UV to induce mutations and obtain mutant strain N5; mutant strain N5 is further irradiated with UV to induce mutations; the resulting mutant strain is subjected to Percoll density gradient centrifugation, after which a low-density fraction is collected and cells from this fraction are cultured; and the fractionation and culturing steps are repeated.

## Description

### Technical Field

The present invention relates to a novel high oil-producing strain belonging to the genus *Lipomyces* and a method of producing an oil using the high oil-producing strain.

### Background Art

Palm oil is a vegetable oil derived from the fruit of the oil palm and is solid at room temperature. Palm oil is characterized by containing a high level of saturated fatty acids, with palmitic acid and oleic acid constituting approximately 80% of total fatty acids. Palm oil is known to be used not only as an edible oil but as a raw material for margarine, shortening, or soaps. Palm oil is also used as a fried oil for instant fried noodles or snack products such as potato chips.

Palm oil is the most widely produced vegetable oil in the world. In 2015, a total of 6256 × 10⁴ tons of palm oil were produced worldwide. The oil palm is a plant that grows in tropical areas with hot and humid climates and is native to West Africa and Latin America. Nowadays, Malaysia and Indonesia account for 80% or more of palm oil production. Since the oil palm bears fruit year-round, its fruit yield is much higher than that of other vegetable oil sources, and palm oil can be produced in quantities 8 to 10 times greater than soybean oil or rapeseed oil. Hence, palm oil is inexpensive compared to other vegetable oils and can be supplied stably, which is why many countries import it.

The oil palm grows exclusively in equatorial tropical regions with hot and humid climates. These regions offer growing conditions suitable for the species but coincide with areas where tropical rainforests are distributed. Thus, developing an oil palm plantation inevitably involves cutting down a tropical rainforest, and many tropical rainforests are cleared and lost every year. Massive forest fires have also occurred during the development of oil palm plantations. Due to such a loss of tropical rainforests, rare species of wild animals inhabiting there are now on the verge of extinction.

Furthermore, a rapid increase in demand for palm oil has led to workers being placed under poor working conditions or sparked conflicts over land development between local residents and developers. Thus, palm oil has caused labor problems and human rights problems as well as global environmental problems.

Against this background, methods of producing an alternative to palm oil using microorganisms have been sought in recent years. Oleaginous yeasts belonging to the genus *Lipomyces* are known to produce oils with fatty acid compositions similar to that of palm oil. In addition, the oils produced by *Lipomyces* yeasts resemble palm oil in physical properties and show promise as a palm oil alternative in terms of environment risk, climate change risk, and sustainability.

In a method of producing an alternative oil using wild-type *Lipomyces starkeyi* as an oleaginous yeast, the amount of the oil produced by the yeast is insufficient. To address this issue, an attempt has been made to mutate the wild-type strain and thus obtain a high oil-producing strain (high oil-accumulating strain) that produces large quantities of oil. Non-Patent Literature 1 or 2 discloses inducing mutations in CBS1807, which is a wild-type strain of *Lipomyces starkeyi,* by means of ethyl methanesulfonate or UV irradiation and repeatedly concentrating the high oil-producing mutant strain through density gradient centrifugation to obtain a concentrated fraction of the high oil-producing strain.

Patent Literature 1 discloses a method of producing a high oil-producing strain by using an oleaginous yeast belonging to the genus *Lipomyces* or *Rhodosporidium* and subjecting the oleaginous yeast to density gradient centrifugation using a reagent consisting of colloidal silicate particles coated with polyvinylpyrrolidone. Patent Literature 1 further discloses treating the yeast using ethyl methanesulfonate or UV as a mutagen and then subjecting the treated yeast to density gradient centrifugation using a reagent consisting of colloidal silicate particles coated with polyvinylpyrrolidone.

### Citation List

### Patent Literature

PTL 1: Japanese Patent No. 7082340

### Non-Patent Literature

NPL 1: Yamazaki H, Kobayashi A, Ebina S, et al. (2019). Highly selective isolation and characterization of Lipomyces starkeyi mutants with increased production of triacylglycerol. Applied Microbiology and Biotechnology. 103, 6297-6308.
NPL 2: Hiroaki Takaku et al., Isolation and characterization of Lipomyces starkeyi mutants with greatly increased lipid productivity following UV irradiation, J Biosci Bioeng., 131(6), 613-621, (2021)

### Summary of Invention

### Technical Problem

The present inventors successfully obtained strain N5 as a high oil-producing strain by inducing mutations in *Lipomyces Starkeyi* CBS1807, a wild-type strain, through UV irradiation. However, the industrial use of strain N5 is disadvantageous in terms of oil production rate and oil conversion efficiency. An object of the present invention is to obtain a high oil-producing strain of yeast belonging to the genus *Lipomyces,* the high oil-producing strain being capable of producing (accumulating) larger quantities of oil than strain N5, and to provide a method of producing a palm oil alternative using the high oil-producing strain.

### Solution to Problem

The present inventors focused on the potential of the method used to obtain strain N5 as a high oil-producing strain from *Lipomyces Starkeyi* CBS1807, a wild-type strain, and investigated whether applying this method using strain N5 as the parental strain could yield a novel mutant strain with increased oil productivity. As a result, the inventors have completed the present invention.

Specifically, the present invention relates to a high oil-producing strain of yeast belonging to the genus *Lipomyces,* the high oil-producing strain being deposited at the National Institute of Technology and Evaluation, Patent Microorganisms Depositary, under accession number NITE BP-03796.

The high oil-producing strain (strain N5-15 described later), which is deposited at the National Institute of Technology and Evaluation, Patent Microorganisms Depositary, under accession number NITE BP-03796, is obtained as follows: strain N5 is irradiated with UV to induce mutations; the resulting mutant strain is subjected to Percoll density gradient centrifugation, after which the upper layer is collected as a low-density fraction and cells from this fraction are cultured; the fractionation and culturing steps are repeated to concentrate the high oil-producing strain; and the strain is then isolated and screened.

The present invention further relates to a method of producing an oil, the method including using a high oil-producing strain of oleaginous yeast belonging to the genus *Lipomyces,* the high oil-producing strain being deposited at the National Institute of Technology and Evaluation, Patent Microorganisms Depositary, under accession number NITE BP-03796.

### Advantageous Effects of Invention

The oil-producing yeast of the present invention can produce a palm oil alternative more efficiently than conventional oil-producing yeasts belonging to the genus *Lipomyces.* Consequently, the oil production method of the present invention can yield a palm oil alternative at lower cost than conventional production methods using known oil-producing yeasts.

### Brief Description of Drawings

FIG. 1 shows images illustrating how cell layers change after density gradient centrifugation. "WT" denotes a wild-type strain, "K14" denotes strain K14, and "UV" denotes UV-treated cells of the wild-type strain.
FIG. 2 shows microscopic images of strains N1 to N9 and control strains.
FIG. 3 is a graph showing the cell concentrations of strains N1, N3, N5, and N8 and the control strains over the three days of culture.
FIG. 4 is a graph showing the average cell size measured over time for the various strains.
FIG. 5 shows microscopic images of the various strains captured on the third day of culture.
FIG. 6 is a graph showing the glucose concentration in medium measured over time for the various strains.
FIG. 7 is a graph showing the triglyceride (TAG) content per volume of medium measured over time for the various strains.
FIG. 8 is a graph showing the triglyceride (TAG) content normalized to cell count, measured over time for the various strains.
FIG. 9 shows images illustrating how cell layers change after density gradient centrifugation. "WT" denotes a wild-type strain, "N5" denotes strain N5, and "UV" denotes UV-treated cells of strain N5.
FIG. 10 is a graph showing the cell concentration measured on the third day of culture for strains N5-1 to N5-16.
FIG. 11 is a graph showing the glucose consumption measured on the third day of culture for strains N5-1 to N5-16 and the control strains.
FIG. 12 is a graph showing the triglyceride (TAG) content per volume of medium measured on the third day of culture for strains N5-1 to N5-16 and the control strains.
FIG. 13 is a graph showing the triglyceride (TAG) content normalized to cell count, measured on the third day of culture for strains N5-1 to N5-16.
FIG. 14 is a graph showing the cell concentrations of strains N5-2, N5-13, and N5-15 and the control strains over the six days of culture.
FIG. 15 is a graph showing the average cell sizes of strains N5-2, N5-13, and N5-15 and the control strains over the six days of culture.
FIG. 16 shows microscopic images of the various strains captured on the sixth day of culture.
FIG. 17 is a graph showing the glucose concentration in medium measured over the six days of culture for strains N5-2, N5-13, and N5-15 and the control strains.
FIG. 18 is a graph showing the triglyceride (TAG) content per volume of medium measured over the six days of culture for strains N5-2, N5-13, and N5-15 and the control strains.
FIG. 19 is a graph showing the triglyceride (TAG) content normalized to cell count, measured over the six days of culture for strains N5-2, N5-13, and N5-15 and the control strains.

### Description of Embodiments

Hereinafter, embodiments of the present invention will be described with reference to the drawings. The present invention is not limited by the following description.

### <Acquisition of high oil-accumulating mutant strains>

### 1. Development of strain N5 from wild-type strain

*Lipomyces starkeyi* CBS1807 (wild-type strain), a yeast belonging to the genus *Lipomyces,* was irradiated with UV to induce mutations. The resulting mutant strain was subjected to Percoll density gradient centrifugation, after which the upper layer was collected as a low-density fraction and cells from this fraction were cultured. The fractionation and culturing steps were repeated to concentrate the high oil-producing strain, and the strain was isolated and screened.

### [Induction of mutations by UV irradiation]

A volume of 3 mL of YPD medium (Table 1) was added to a sterile test tube and inoculated with strain *Lipomyces starkeyi* CBS1807 from a stock plate. The unit of the final concentration shown in Table 1 is w/v%, and the same applies to Tables 2 to 6 referenced later. The cells of the strain were cultured at 30°C for two days (preculture), after which the culture fluid was collected. The collected culture fluid was adjusted to a cell concentration of 1.0 × 10⁷ cells/mL using YPD medium, and 7 mL of the culture fluid was transferred to an L-shaped test tube, in which the cells were cultured for 24 hours (main culture). Subsequently, the entire main culture fluid was transferred to a 15-mL tube and centrifuged at 2270 × g for five minutes, after which the supernatant was removed.

**[Table 1]**

| | Final concentration |
|---|---|
| Yeast extract | 1% |
| HIPOLYPEPTON | 2% |
| D-(+)-Glucose | 2% |

The precipitated cells were washed by suspending them in 7 mL of 1 × PBS, centrifuging the suspension at 2270 × g for five minutes, and then removing the supernatant. After that, 7 mL of 1 × PBS was added to an L-shaped test tube, and the cells were suspended in the PBS. The suspension was diluted to a cell concentration of 1.0 × 10⁷ cells/mL using 1 × PBS, and 10 mL of the cell suspension was dispensed into a sterile Petri dish.

Next, the Petri dish containing the cell suspension was placed at a distance of 25 cm from the UV tube of a UV irradiator (TOSHIBA Lighting & Technology Corporation, model GL15, output = 4.9 W), and the cell suspension was irradiated with UV for 20 seconds. The UV irradiation energy is calculated, for example, as 0.816 (mW/cm²) × 20 (sec) = 16.32 (mJ/cm²) in the case of the 20-second irradiation. After the UV irradiation, the cells in the Petri dish were cultured at 30°C for two days. The entire cell suspension in the Petri dish was added to a 15-mL tube and centrifuged at 2270 × g for five minutes, after which the supernatant was removed. The cells were washed by adding 1 mL of SG medium (Table 2) to the tube to suspend the cells, centrifuging the suspension at 2270 × g for five minutes, and removing the supernatant.

**[Table2]**

| | Final concentration |
|---|---|
| (NH₄)₂SO₄ | 0.5% |
| KH₂PO₄ | 0.1% |
| NaCl | 0.01% |
| Yeast extract | 0.1% |
| Glycerol | 2% |
| MgSO₄•7H₂O | 0.05% |
| CaCl₂•7H₂O | 0.01% |

To the tube was added 7 mL of SG medium to suspend the cells. The entire culture fluid in the tube was used to inoculate a sterile L-shaped test tube, in which the cells were cultured at 30°C and 120 rpm for three days (initial culture). The initial culture fluid was adjusted to a final cell concentration of 1.25 × 10⁶ cells/mL using S5% medium (Table 3), and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for two days (main culture).

**[Table 3]**

| | Final concentration |
|---|---|
| (NH₄)₂SO₄ | 0.5% |
| KH₂PO₄ | 0.1% |
| NaCl | 0.01% |
| Yeast extract | 0.1% |
| D-(+)-Glucose | 5% |
| MgSO₄•7H₂O | 0.05% |
| CaCl₂•7H₂O | 0.01% |

### [Concentration of high oil-producing mutant strains by density gradient centrifugation using Percoll reagent]

The number of cells in the main culture fluid was determined by means of a cell counter, and a volume containing 1.0 × 10⁹ cells was transferred to a 15-mL tube. A suspension of *Lipomyces starkeyi* CBS1807 (wild-type strain) having undergone main culture and a suspension of strain K14 (Non-Patent Literature 1) having undergone main culture were also added to tubes.

Each tube was centrifuged (swing rotor, 2270 × g, five minutes, room temperature), and the supernatant was removed. To the tube was added 500 µL of sterile 1 × PBS to suspend the cells, and the entire suspension was transferred to a 1.5-mL tube. After that, 1 × PBS was added to bring the suspension volume to the 1.0-mL mark on the 1.5-mL tube.

A volume of 8 mL of Percoll reagent (concentration = 40%) was added to an ultracentrifugation tube, to which the entire cell suspension was transferred from the 1.5-mL tube. The ultracentrifugation tube was gently inverted to mix its contents, and the mixture was subjected to ultracentrifugation using an ultracentrifuge (70.1Ti rotor, 22000 rpm, 20 minutes, room temperature, ACCEL SLOW, DECEL NO BREAK). After the ultracentrifugation, the cell layer formed was observed. Subsequently, 1 mL of the upper layer formed as a low-density fraction was transferred to a 15-mL tube by means of a peristaltic pump.

The cells were washed as follows: sterile 1 × PBS whose volume was five times that of the transferred upper layer was added to the tube to suspend the cells, and the suspension was centrifuged at 2270 × g for five minutes, after which the supernatant was removed. The cells were further washed by adding 1 mL of SG medium to the tube to suspend the cells, centrifuging the suspension at 2270 × g for five minutes, and removing the supernatant. To the tube was added 7 mL of SG medium to suspend the cells.

### [Cell culture]

The entire culture fluid in the tube was used to inoculate a sterile L-shaped test tube, in which the cells were cultured at 30°C and 120 rpm for three days (initial culture). The initial culture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for two days (main culture).

### [Concentration of high oil-producing mutant strains by density gradient centrifugation using Percoll reagent: second round]

The procedures described in [Concentration of high oil-producing mutant strains by density gradient centrifugation using Percoll reagent] through [Cell culture] were repeated.

### [Concentration of high oil-producing mutant strains by density gradient centrifugation using Percoll reagent: third round]

The procedures described in [Concentration of high oil-producing mutant strains by density gradient centrifugation using Percoll reagent] through [Cell culture] were further repeated. The main culture in [Cell culture] was carried out for three days.

### [Concentration of high oil-producing mutant strains by density gradient centrifugation using Percoll reagent: fourth round]

The procedures described in [Concentration of high oil-producing mutant strains by density gradient centrifugation using Percoll reagent] through [Cell culture] were repeated once more. After that, 1 mL of the main culture fluid was added to a tube, and the cells were washed by adding sterile 1 × PBS whose volume was five times that of the main culture fluid to suspend the cells, centrifuging the suspension at 2270 × g for five minutes, and removing the supernatant. Next, the cells were further washed by adding 1 mL of S5% medium to the tube to suspend the cells, centrifuging the suspension at 2270 × g for five minutes, and removing the supernatant.

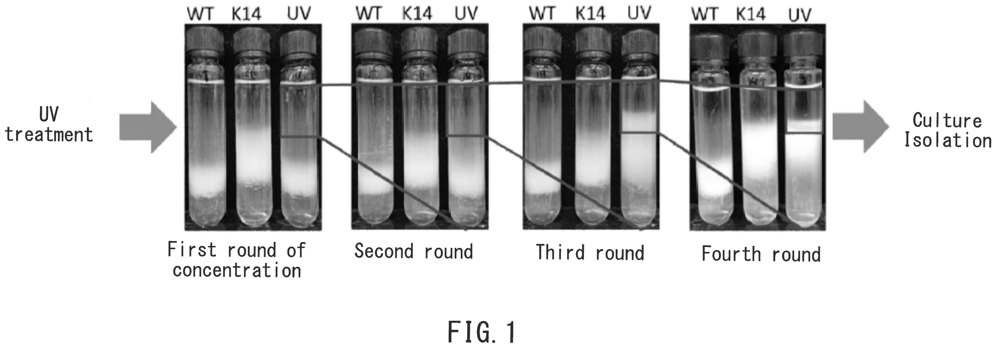

When low-density cells that have produced and accumulated large quantities of oil are repeatedly concentrated, it is thought that the cell layer will gradually shift to a low-density fraction. As a result, the cell layer is expected to appear at a higher position than that of the wild-type strain or strain K14 after repeated rounds of concentration. After the first concentration process, as shown in FIG. 1, the cell layer of the UV-treated sample was not observed above the cell layer of strain K14 used as a control. After the second round of concentration, the cell layer of the UV-treated sample was found at a higher position than the upper portion of the cell layer of strain K14. The third and fourth rounds of concentration rendered the cell layer of the UV-treated sample clearly identifiable. This indicates that high oil-producing cells were more concentrated in the case of the UV-treated sample than in the case of strain K14. In FIG. 1, "WT" denotes the wild-type strain. The same applies to the other figures referenced later.

### [Isolation of strains]

Colony isolation was carried out as follows: 100 µL of S5% medium was added to the tube to suspend the cells and then the suspension was plated onto S5% solid medium (Table 4), on which the cells were cultured at 30°C for three days. It has been found that *Lipomyces starkeyi* produces an acidic polysaccharide containing mannose, galactose, glucuronic acid, and a small amount of glucose as constituent sugars and that cells producing this polysaccharide form a mucoid colony. Thus, strains prone to convert glucose (carbon source) taken from the medium into the polysaccharide are thought to have low oil productivity. With this in mind, colonies that did not have any shiny appearance indicative of polysaccharide production were separated from the plate and re-streaked on S5% medium, on which the cells were cultured at 30°C for three days. Among the cells of the re-streaked colonies, those having a shiny appearance and thought to have produced the polysaccharide were removed to obtain nine strains N1 to N9.

**[Table 4]**

| | Final concentration |
|---|---|
| (NH₄)₂SO₄ | 0.5% |
| KH₂PO₄ | 0.1% |
| NaCl | 0.01% |
| Yeast extract | 0.1% |
| D-(+)-Glucose | 5% |
| MgSO₄•7H₂O | 0.05% |
| CaCl₂•7H₂O | 0.01% |
| Agar | 2% |

### [Primary screening of high oil-producing strains by microscopic observation]

L-shaped test tubes containing 7 mL of SG medium were inoculated with the colonies of the nine strains N1 to N9, the wild-type strain serving as a negative control, and strain K14 serving as a positive control, and the cells of the strains were cultured at 30°C and 120 rpm for three days (initial culture). Each initial culture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for three days (main culture).

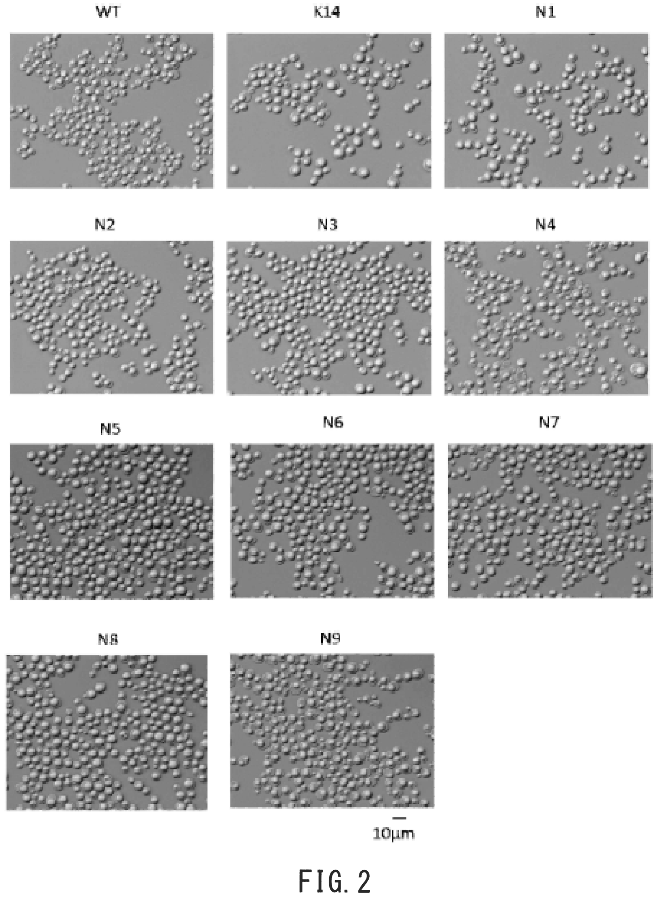

On the third day of main culture, the culture fluids were observed with a microscope to compare the fat globule size between them. Strains N1, N3, N5, and N8 were found to have fat globules as large as or larger than those of strain K14 used as the positive control. These strains N1, N3, N5, and N8 were subjected to secondary screening. FIG. 2 shows microscopic images of strains N1 to N9 and the control strains.

### [Secondary screening of high oil-producing strains by physical and chemical analysis]

L-shaped test tubes containing 7 mL of SG medium were inoculated with the colonies of strains N1, N3, N5, and N8, the wild-type strain serving as a negative control, and strain K14 serving as a positive control, and the cells of the strains were cultured at 30°C and 120 rpm for three days (initial culture). Each initial culture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for three days (main culture).

### [Measurement of cell count]

The cell count (the number of cells) in the medium was measured as follows: The main culture fluid was diluted with a 0.75% saline solution, then ultrasonically treated (output = 20%, on time = 5 sec, off time = 5 sec, two cycles) by means of an ultrasonic homogenizer (Vibra cell of SONICS), and subjected to cell count measurement using CDA-1000 (Sysmex). The average cell size can be simultaneously measured.

### [Measurement of glucose concentration in medium]

A volume of 1 mL of the main culture fluid was transferred to a 1.5-mL tube, and the glucose concentration in the main culture fluid was measured by means of Glucose CII-Test Wako (Wako Pure Chemical Industries, Ltd.). The glucose consumption (g/L) of each mutant strain during culture was calculated based on the measured glucose concentration.

### [Measurement of amount of triglyceride produced]

A volume of the main culture fluid was transferred to a 2-mL tube for Multi-beads Shocker and centrifuged at 2270 × g for five minutes, after which the supernatant was removed. The cells were washed by adding 1 mL of PBS to the tube to suspend the cells, centrifuging the suspension at 2270 × g for five minutes, and removing the supernatant. The tube was heated by means of a heat block at 70°C for five minutes to deactivate the intracellular lipase. The tube was frozen at 30°C, and the cells were freeze-dried by means of a freeze dryer (FDU-1200 of EYELA). To the tube containing the freeze-dried cells were added 500 µL of PBS and 1 g of 0.5-mm glass beads which were washed with 0.1N hydrochloric acid and dried in advance. The cells were then disrupted (2500 rpm, 900 seconds) by means of Multi-beads Shocker (Yasui Kikai Corporation). A volume of 500 µL of 1 × PBS was added to the tube, and the tube was shaken by means of a mixer (Micromixer E-36 of Taitec Corporation) at 37°C for 10 minutes. The sample thus obtained was used as a sample for triglyceride quantification. To a 1.5-mL tube were added 1 mL of chromogenic reagent of Triglyceride E-Test Wako (FUJIFILM Wako Pure Chemical Corporation) and 6.7 µL of the sample for triglyceride quantification, and the contents of the tube were mixed by gentle inversion. The tube was heated by means of a heat block at 37°C for 10 minutes while the tube was gently inverted every two minutes. After the heating, the absorbance of the sample was measured at 600 nm. The chromogenic reagent heated in the same manner as the sample was used as a control in the measurement, and the triglyceride concentration in the sample was determined from the calibration curve.

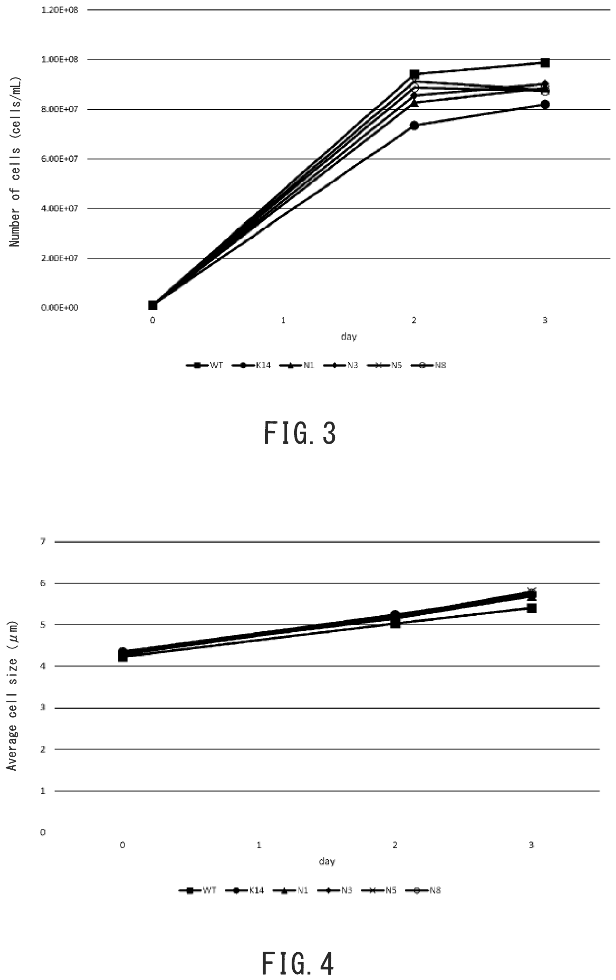

FIG. 3 is a graph showing the cell concentration measured over time for strains N1, N3, N5, and N8 and the control strains during the three days of culture. The difference in growth rate was observed already in the early stage of culture, and the growth rate and the final cell concentration were lower for strains N1, N3, N5, and N8 than for the wild-type strain. On the third day of culture, the cell concentration of strain N1 was 0.90 times that of the wild-type strain, the cell concentration of strain N3 was 0.91 times that of the wild-type strain, the cell concentration of strain N5 was 0.89 times that of the wild-type strain, and the cell concentration of strain N8 was 0.89 times that of the wild-type strain.

FIG. 4 is a graph showing the average cell size measured over time for strains N1, N3, N5, and N8 and the control strains during the three days of culture. On the fourth day of culture, the average cell size of strain N1 was about 1.05 times that of the wild-type strain, the average cell size of strain N3 was about 1.06 times that of the wild-type strain, the average cell size of strain N5 was about 1.07 times that of the wild-type strain, and the average cell size of strain N8 was about 1.07 times that of the wild-type strain.

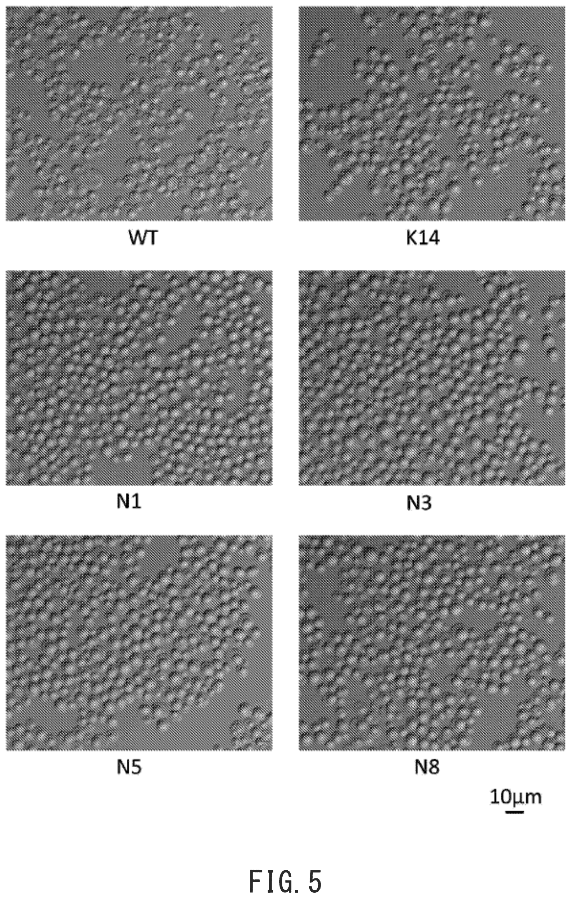

FIG. 5 shows microscopic images of the various strains captured on the third day of culture. As seen from FIG. 5, strains N1, N3, N5, and N8 were found to have larger fat globules than the wild-type strain. This demonstrates that strains N1, N3, N5, and N8 are superior in oil productivity.

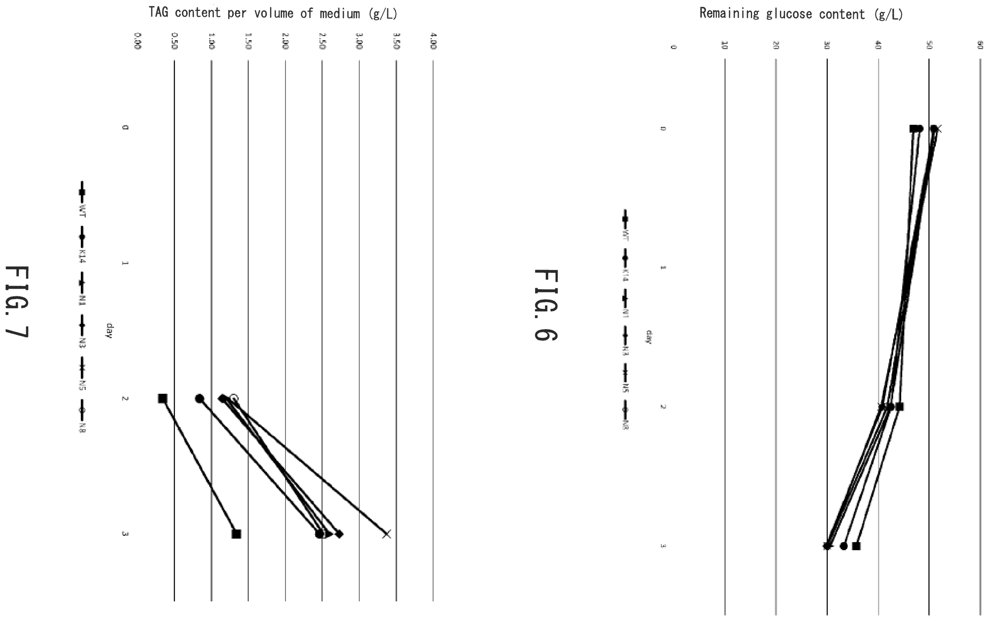

FIG. 6 is a graph showing the remaining glucose content (glucose concentration) in medium measured over time for the various strains. As seen from FIG. 6, on the third day of culture, strains N1, N3, N5, and N8 were found to have assimilated a larger amount of glucose from the medium than the wild-type strain.

FIG. 7 is a graph showing the triglyceride (TAG) content per volume of medium measured over time for the various strains. As seen from FIG. 7, on the third day of culture, the triglyceride content per volume of medium for strain N1 was about 1.93 times that for the wild-type strain, the triglyceride content per volume of medium for strain N3 was about 2.03 times that for the wild-type strain, the triglyceride content per volume of medium for strain N5 was about 2.51 times that for the wild-type strain, and the triglyceride per volume of medium for strain N8 was about 1.87 times that for the wild-type strain.

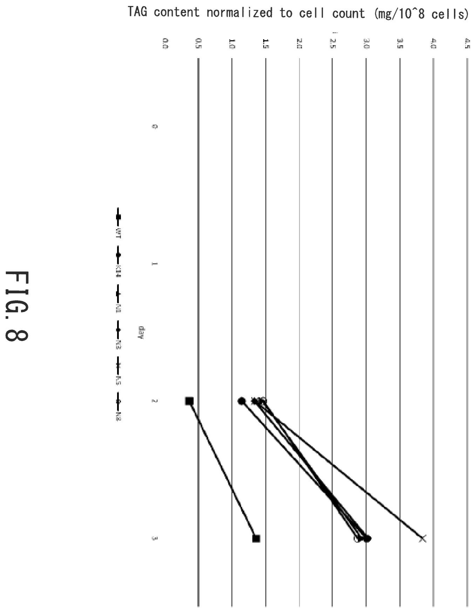

FIG. 8 is a graph showing the triglyceride (TAG) content normalized to cell count, measured over time for the various strains. As seen from FIG. 8, on the third day of culture, the triglyceride content normalized to cell count for strain N1 was about 2.16 times that for the wild-type strain, the triglyceride content normalized to cell count for strain N3 was about 2.23 times that for the wild-type strain, the triglyceride content normalized to cell count for strain N5 was about 2.83 times that for the wild-type strain, and the triglyceride content normalized to cell count for strain N8 was about 2.11 times that for the wild-type strain.

### 2. Development of strain N5-15 from strain N5

Strain *Lipomyces starkeyi* N5 obtained as a high oil-producing strain by the procedures described above (the mutant strain that produced the largest amount of triglyceride) was further irradiated with UV to induce mutations and thus develop a novel mutant strain as another high oil-producing strain. The method employed was the same as that used for the development of strain N5 from the wild-type strain. Specifically, the mutant strain was subjected to Percoll density gradient centrifugation, after which the upper layer was collected as a low-density fraction and cells from this fraction were cultured. The fractionation and culturing steps were repeated to concentrate the high oil-producing strain, and the strain was isolated and screened.

### [Induction of mutations by UV irradiation]

A volume of 3 mL of YPD medium was added to a sterile test tube and inoculated with strain *Lipomyces starkeyi* N5 from a stock plate. The cells of the strain were cultured at 30°C for two days (preculture). The culture fluid was collected and then adjusted to a cell concentration of 1.0 × 10⁷ cells/mL using YPD medium, and 7 mL of the culture fluid was transferred to an L-shaped test tube, in which the cells were cultured for 24 hours (main culture). Subsequently, the entire main culture fluid was transferred to a 15-mL tube and centrifuged at 2270 × g for five minutes, after which the supernatant was removed.

The precipitated cells were washed by suspending them in 7 mL of 1 × PBS, centrifuging the suspension at 2270 × g for five minutes, and then removing the supernatant. After that, 7 mL of 1 × PBS was added to an L-shaped test tube, and the cells were suspended in the PBS. The suspension was diluted to a cell concentration of 1.0 × 10⁷ cells/mL using 1 × PBS, and 10 mL of the cell suspension was dispensed into a sterile Petri dish.

Next, the Petri dish containing the cell suspension was placed at a distance of 25 cm from the UV tube of a UV irradiator (TOSHIBA Lighting & Technology Corporation, model GL15, output = 4.9 W), and the cell suspension was irradiated with UV for 25 seconds. After the UV irradiation, the cells in the Petri dish were cultured at 30°C for two days. The entire cell suspension in the Petri dish was added to a 15-mL tube and centrifuged at 2270 × g for five minutes, after which the supernatant was removed. The cells were washed by adding 1 mL of SG medium to the tube to suspend the cells, centrifuging the suspension at 2270 × g for five minutes, and removing the supernatant.

To the tube was added 7 mL of SG medium to suspend the cells. The entire culture fluid in the tube was used to inoculate a sterile L-shaped test tube, in which the cells were cultured at 30°C and 120 rpm for three days (initial culture). The initial culture fluid was adjusted to a final cell concentration of 1.25 × 10⁶ cells/mL using S7% medium (Table 5), and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S7% medium, and a 200-mL baffled flask was inoculated with the culture fluid at a scale of 75 mL. The cells were then cultured in the flask at 30°C and 160 rpm for two days (main culture).

**[Table 5]**

| | Final concentration |
|---|---|
| (NH₄)₂SO₄ | 0.5% |
| KH₂PO₄ | 0.1% |
| NaCl | 0.01% |
| Yeast extract | 0.1% |
| D-(+)-Glucose | 7% |
| MgSO₄•7H₂O | 0.05% |
| CaCl₂•7H₂O | 0.01% |

### [Concentration of high oil-producing mutant strains by density gradient centrifugation using Percoll reagent]

The number of cells in the main culture fluid was determined by means of a cell counter, and a volume containing 1.0 × 10⁹ cells was transferred to a 15-mL tube. A suspension of *Lipomyces starkeyi* CBS1807 (wild-type strain) having undergone main culture and a suspension of strain N5 having undergone main culture were also added to tubes.

Each tube was centrifuged (swing rotor, 2270 × g, 5 minutes, room temperature), and the supernatant was removed. To the tube was added 500 µL of sterile 1 × PBS to suspend the cells, and the entire suspension was transferred to a 1.5-mL tube. After that, 1 × PBS was added to bring the suspension volume to the 1.0-mL mark on the 1.5-mL tube.

A volume of 8 mL of Percoll reagent (concentration = 40%) was added to an ultracentrifugation tube, to which the entire cell suspension was transferred from the 1.5-mL tube. The ultracentrifugation tube was gently inverted to mix its contents, and the mixture was subjected to ultracentrifugation using an ultracentrifuge (70.1Ti rotor, 22000 rpm, 20 minutes, room temperature, ACCEL SLOW, DECEL NO BREAK). After the ultracentrifugation, the cell layer formed was observed. Subsequently, 1 mL of the upper layer formed as a low-density fraction was transferred to a 15-mL tube by means of a peristaltic pump.

The cells were washed as follows: sterile 1 × PBS whose volume was five times that of the transferred upper layer was added to the tube to suspend the cells, and the suspension was centrifuged at 2270 × g for five minutes, after which the supernatant was removed. The cells were further washed by adding 1 mL of SG medium to the tube to suspend the cells, centrifuging the suspension at 2270 × g for five minutes, and removing the supernatant. To the tube was added 7 mL of SG medium to suspend the cells.

### [Cell culture]

The entire culture fluid in the tube was used to inoculate a sterile L-shaped test tube, in which the cells were cultured at 30°C and 120 rpm for three days (initial culture). The initial culture fluid was adjusted to a final cell concentration of 1.25 × 10⁶ cells/mL using S7% medium, and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S7% medium, and a 200-mL baffled flask was inoculated with the culture fluid at a scale of 75 mL. The cells were then cultured in the flask at 30°C and 160 rpm for two days (main culture).

### [Concentration of high oil-producing mutant strains by density gradient centrifugation using Percoll reagent: second round]

The procedures described in [Concentration of high oil-producing mutant strains by density gradient centrifugation using Percoll reagent] through [Cell culture] were repeated.

### [Concentration of high oil-producing mutant strains by density gradient centrifugation using Percoll reagent: third round]

The procedures described in [Concentration of high oil-producing mutant strains by density gradient centrifugation using Percoll reagent] through [Cell culture] were further repeated. The cells in the tube were washed by adding sterile 1 × PBS whose volume was five times that of the main culture fluid to suspend the cells, centrifuging the suspension at 2270 × g for five minutes, and removing the supernatant. Next, the cells were further washed by adding 1 mL of S7% medium to the tube to suspend the cells, centrifuging the suspension at 2270 × g for five minutes, and removing the supernatant.

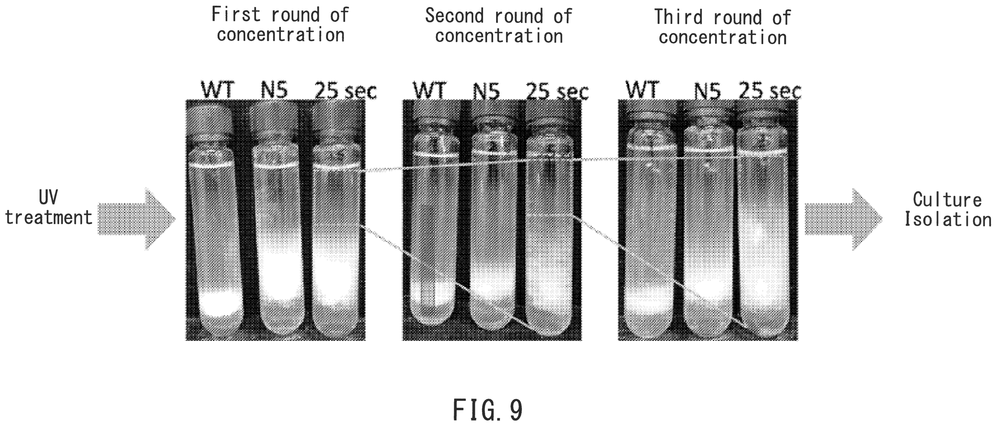

When low-density cells that have produced and accumulated large quantities of oil are repeatedly concentrated, it is thought that the cell layer will gradually shift to a low-density fraction. As a result, the cell layer is expected to appear at a higher position than that of the wild-type strain or strain N5 after repeated rounds of concentration. FIG. 9 shows images illustrating how the cell layers change after density gradient centrifugation. After the first concentration process, as shown in FIG. 9, the cell layer of the UV-treated sample was not observed above the cell layer of strain N5 used as a control. After the second round of concentration, the cell layer of the UV-treated sample was found at a higher position than the upper portion of the cell layer of strain N5. The third round of concentration rendered the cell layer of the UV-treated sample clearly identifiable. This indicates that high oil-producing cells were more concentrated in the case of the UV-treated sample than in the case of strain N5.

### [Isolation of strains]

Colony isolation was carried out as follows: 100 µL of S7% medium was added to the tube to suspend the cells and then the suspension was plated onto S7% solid medium (Table 6), on which the cells were cultured at 30°C for three days. Non-mucoid colonies were re-streaked on S5% medium, on which the cells were cultured at 30°C for three days. Among the cells of the re-streaked colonies, those having a shiny appearance and thought to have produced a polysaccharide were removed to obtain 16 strains N5-1 to N5-16.

**[Table 6]**

| | Final concentration |
|---|---|
| (NH₄)₂SO₄ | 0.5% |
| KH₂PO₄ | 0.1% |
| NaCl | 0.01% |
| Yeast extract | 0.1% |
| D-(+)-Glucose | 7% |
| MgSO₄•7H₂O | 0.05% |
| CaCl₂•7H₂O | 0.01% |
| Agar | 2% |

### [Primary screening of high oil-producing strains]

L-shaped test tubes containing 7 mL of SG medium were inoculated with the colonies of the 16 strains N5-1 to N5-16 and the colonies of the wild-type strain and strain N5 serving as controls, and the cells of the strains were cultured at 30°C and 120 rpm for three days (initial culture). Each initial culture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for 24 hours (preculture). The preculture fluid was adjusted to a final cell concentration of 1.25 × 10⁶ cells/mL using S5% medium, and a 200-mL baffled flask was inoculated with the culture fluid at a scale of 75 mL. The cells were then cultured in the flask at 30°C and 160 rpm for three days (main culture).

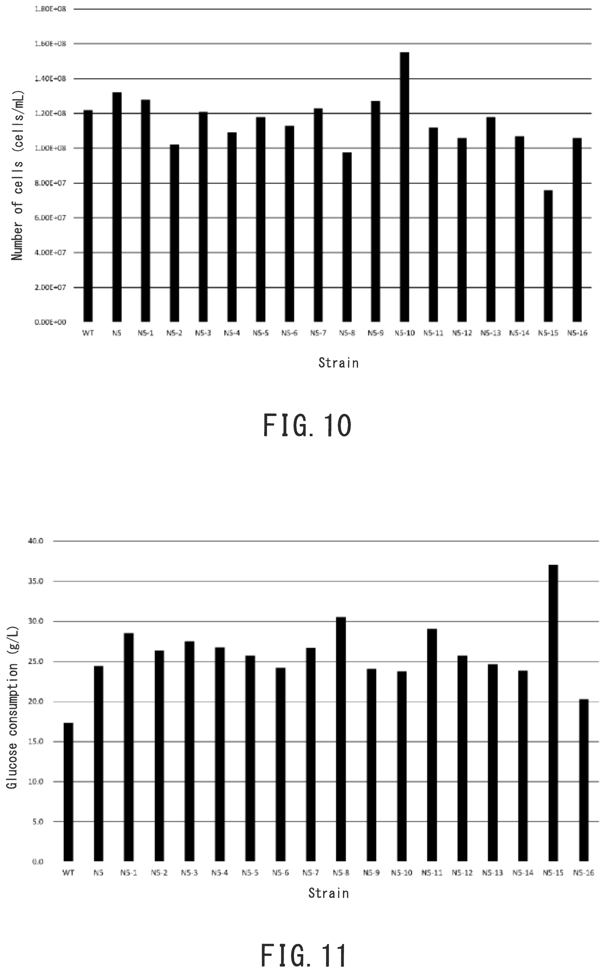

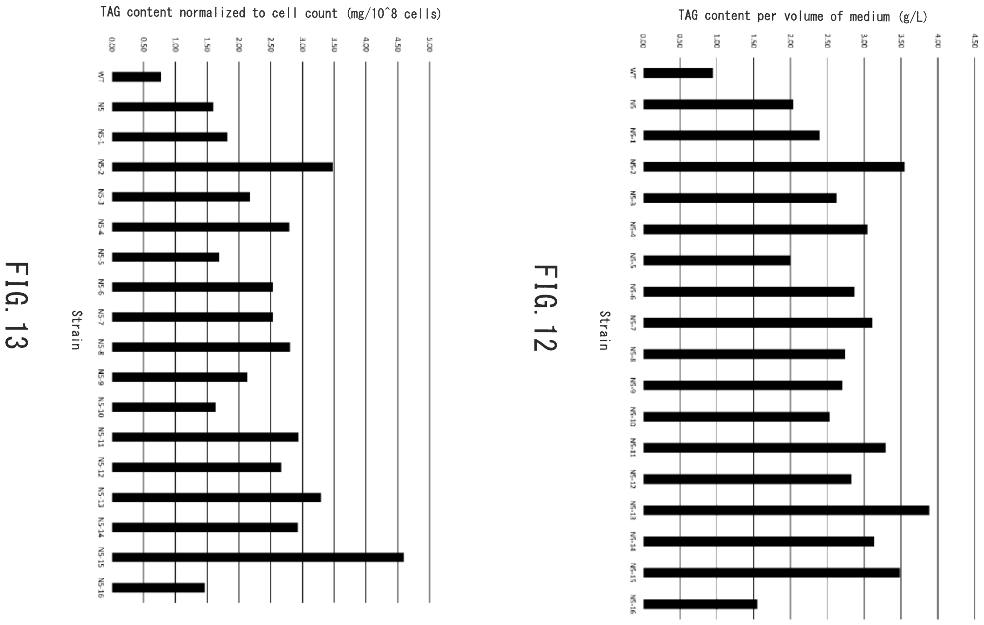

A volume of each culture fluid was collected on the third day of main culture and analyzed for cell concentration (the number of cells, FIG. 10), glucose consumption (FIG. 11), amount of triglyceride produced per volume of medium (FIG. 12), and amount of produced triglyceride normalized to cell count (FIG. 13) in the same manner as in "1. Development of strain N5 from wild-type strain". As seen from FIGS. 10 to 13, strains N5-2, N5-13, and N5-15 were observed to produce a significantly larger amount of triglyceride than strain N5. Thus, these three strains were subjected to secondary screening.

### [Secondary screening of high oil-producing strains]

A volume of 50 mL of SG medium was added to individual 200-mL baffled flasks, which were inoculated with the colonies of strains N5-2, N5-13, N5-15 and the colonies of the wild-type strain and strain N5 serving as controls. The cells in the flasks were cultured at 30°C and 160 rpm for three days (initial culture). Each initial culture fluid was adjusted to a final cell concentration of 3.0 × 10⁶ cells/mL using S7% medium, and a 500-mL baffled flask was inoculated with the culture fluid at a scale of 200 mL. The cells were then cultured in the flask at 30°C and 160 rpm for 24 hours (preculture). The preculture fluid was adjusted to a cell concentration of 1.2 × 10⁷ cells/mL using S7% medium, and a 500-mL baffled flask was inoculated with the culture fluid at a scale of 200 mL. The cells were then cultured in the flask at 30°C and 120 rpm for six days (main culture).

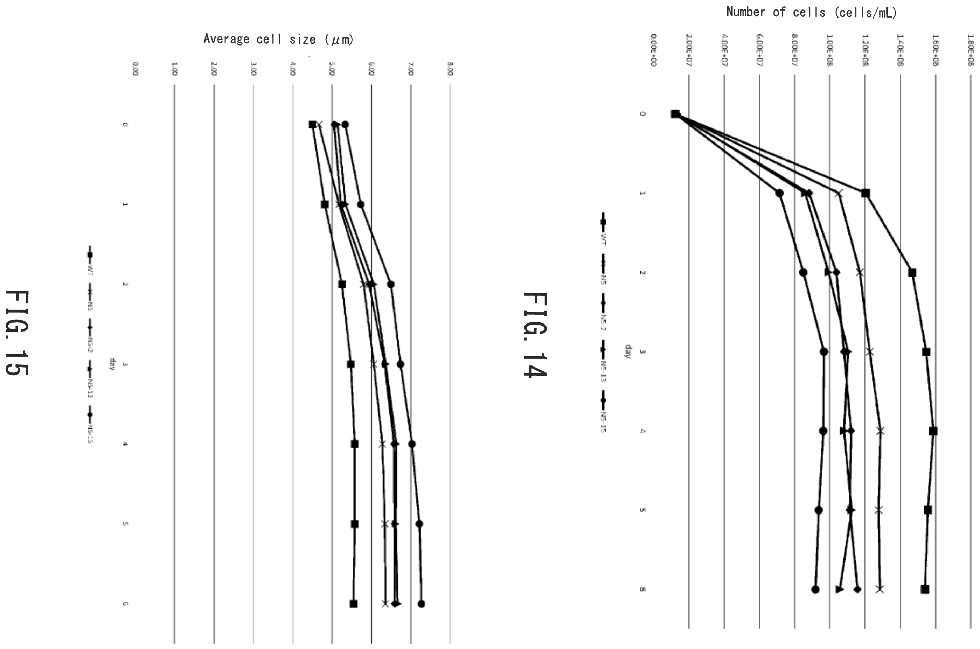

FIG. 14 is a graph showing the cell concentration measured over the six days of culture for strains N5-2, N5-13, and N5-15 and the control strains. The difference in growth rate was observed already in the early stage of culture, and the growth rate and the final cell concentration were lower for strains N5-2, N5-13, and N5-15 than for strain N5. On the third day of culture, the cell concentration of strain N5-2 was 0.88 times that of strain N5, the cell concentration of strain N5-13 was 0.90 times that of strain N5, and the cell concentration of strain N5-15 was 0.79 times that of strain N5.

FIG. 15 is a graph showing the average cell size measured over the six days of culture for strains N5-2, N5-13, and N5-15 and the control stains. For all of strains N5-2, N5-13, and N5-15, the average cell size was already larger than for the wild-type strain on the 0th day of culture. The cells of strains N5-2 and N5-13 reached their maximum size on the fourth day of culture, and the cells of strain N5-15 continued growing until the sixth day. On the fourth day of culture, the cells of strain N5-2 were about 1.05 times larger than those of strain N5, the cells of strain N5-13 were about 1.06 times larger than those of strain N5, and the cells of strain N5-15 were about 1.12 times larger than those of strain N5.

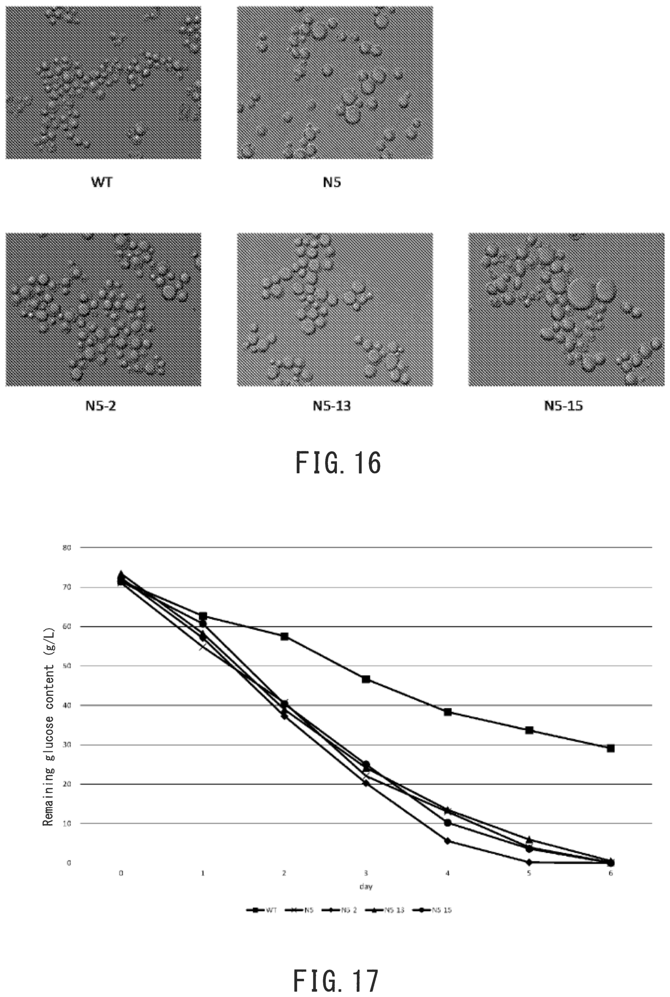

FIG. 16 shows microscopic images of the wild-type strain (WT) and strains N5, N5-2, N5-13, and N5-15 captured on the sixth day of culture. As seen from FIG. 16, the cells of strains N5-2, N5-13, and N5-15 were found to be larger than those of strain N5. In addition, strains N5-2, N5-13, and N5-15 were found to have larger fat globules than strain N5. This demonstrates that strains N5-2, N5-13, and N5-15 are superior in oil productivity.

FIG. 17 is a graph showing the glucose concentration (remaining glucose concentration ) in medium measured over the six days of culture for strains N5-2, N5-13, and N5-15 and the control strains. As seen from FIG. 17, strains N5, N5-2, and N5-13 completely assimilated the glucose in the medium on the sixth day of culture. Strain N5-15 assimilated glucose faster than the other strains and exhausted it between the fifth and sixth days.

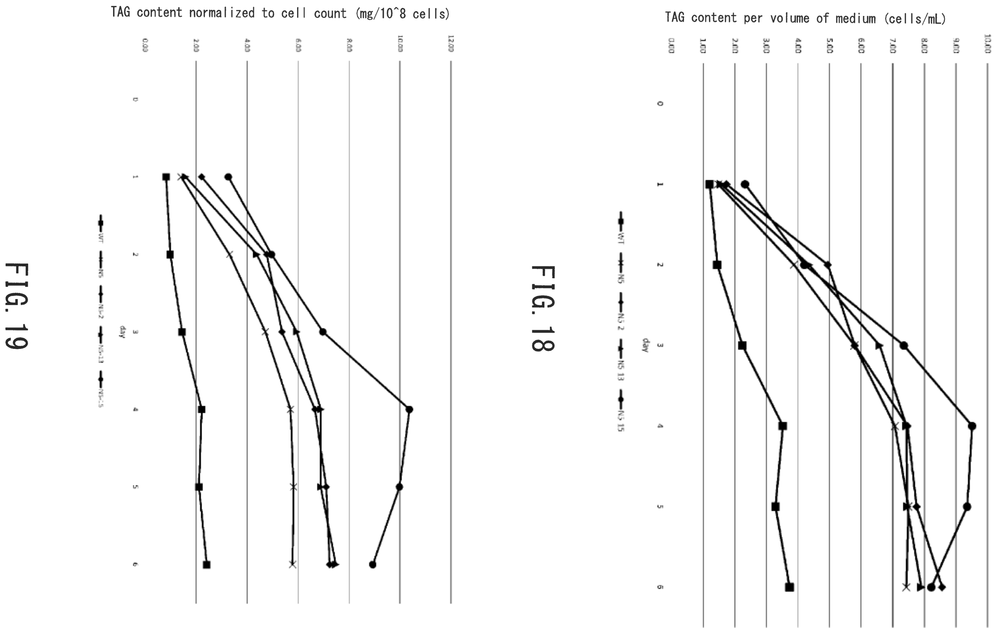

FIG. 18 is a graph showing the triglyceride (TAG) content per volume of medium measured over the six day of culture for strains N5-2, N5-13, and N5-15 and the control strains. As seen from FIG. 18, on the fourth day of culture, the triglyceride content per volume of medium for strain N5-2 was about 1.11 times that for strain N5, the triglyceride content per volume of medium for strain N5-13 was about 1.09 times that for strain N5, and the triglyceride content per volume of medium for strain N5-15 was about 1.35 times that for strain N5.

FIG. 19 is a graph showing the triglyceride content (TAG) normalized to cell count, measured over the six days of culture for strains N5-2, N5-13, and N5-15 and the control strains. As seen from FIG. 19, the triglyceride content normalized to cell count for strain N5-2 was about 1.17 times that for strain N5, the triglyceride content normalized to cell count for strain N5-13 was about 1.21 times that for strain N5, and the triglyceride content normalized to cell count for strain N5-15 was about 1.82 times that for strain N5. The more glucose that was assimilated, the more triglyceride was produced; this indicates a correlation between glucose consumption and triglyceride production. That is, strain N5-15, which exhibited the fastest glucose consumption, produced triglyceride at the highest rate.

### <Analysis of fatty acid composition>

Fatty acid composition was analyzed in the fatty acids produced by the cells of the five strains: N5-2, N5-13, and N5-15, the wild-type strain, and N5.

### [Cell culture]

A volume of 50 mL of SG medium was added to individual 200-mL baffled flasks, which were inoculated with the colonies of strains N5-2, N5-13, N5-15, the wild-type strain, and strain N5. The cells in the flasks were cultured at 30°C and 160 rpm for three days (initial culture). Each initial culture fluid was adjusted to a final cell concentration of 3.0 × 10⁶ cells/mL using S medium, and a 500-mL baffled flask was inoculated with the culture fluid at a scale of 200 mL. The cells were then cultured in the flask at 30°C and 120 rpm for 24 hours (preculture). The preculture fluid was adjusted to a cell concentration of 1.2 × 10⁷ cells/mL using S7% medium, and a 500-mL baffled flask was inoculated with the culture fluid at a scale of 200 mL. The cells were then cultured in the flask at 30°C and 120 rpm for four days (main culture).

### [Samples for fatty acid composition analysis]

A sample for triglyceride quantification was prepared from each main culture fluid in the same manner as in [Measurement of amount of triglyceride produced] of "1. Development of strain N5 from wild-type strain" described above. A tube for Multi-beads Shocker was charged with 125 µL of chloroform, 250 µL of methanol, and 100 µL of the sample for triglyceride quantification, and 0.4 g of glass beads were added (No. 0.40 of Sansho Kenmazai Co., Ltd.). The cells were then disrupted by means of Multi-beads Shocker (2500 rpm, 4°C, 900 seconds).

To the tube were added 125 µL of chloroform and 125 µL of sterile purified water, and then the cells were further disrupted by means of Multi-beads Shocker (2500 rpm, 4°C, 300 seconds). The disrupted cell suspension in the tube was stirred by means of a vortex mixer and transferred to a new 2-mL tube. To the tube were added 200 µL of chloroform, 200 µL of methanol, and 180 µL of sterile purified water, and the contents of the tube were stirred using the vortex mixer.

The tube was centrifuged at 4°C and 10000 × g for five minutes, after which the organic layer formed as a lower layer was separated and transferred to a new 2-mL tube. The solvent was distilled off from the tube using nitrogen gas, and the fatty acids in the tube were converted to methyl esters using a fatty acid methylation kit for triglyceride (Nacalai Tesque, Inc.). The methyl esterified sample was processed into a sample for fatty acid composition analysis according to the instructions for use of the kit. The sample for fatty acid composition analysis was subjected to fatty acid composition analysis using gas chromatography under the conditions listed in Table 7.

**[Table 7]**

| Gas chromatography conditions | |
|---|---|
| System | GC-2014, gas chromatograph manufactured by Shimadzu Corporation |
| Column | DB-23 column, manufactured by Agilent Technologies length = 60 m, inner diameter = 0.25 mm, film thickness = 0.15 µm |
| Injection volume | 1 µL |
| Injection method | Split injection, 10:1 |
| Temperature on the day of injection | 250°C |
| Pressure on the day of injection | 150 kPa |
| Oven | 50°C (1 min), heating to 175°C at 25°C/min, heating to 230°C at 4°C/min |
| Carrier gas | Helium, linear velocity = 22.1 cm/s |
| Column flow rate | 1.11 mL/min |
| Purge flow rate | 3.0 mL/min |
| Total flow rate | 15.2 mL/min |
| Detector | FID, 280°C |
| Detector gas | Hydrogen, air |

Table 8 shows the results of the fatty acid composition analysis of triglyceride produced by the five strains. The fatty acid composition analysis was performed on the fourth day of culture. For strain N5, the palmitic acid content was slightly higher and the stearic acid content was slightly lower than for the wild-type strain (WT). The fatty acid composition determined for each of strains N5-2, N5-13, and N5-15 developed by mutating strain N5 was substantially the same as that determined for strain N5. The fatty acid composition determined for each of these mutant strains consisted mainly of palmitic acid and oleic acid, and an oil with this composition is a promising candidate to serve as an alternative to palm oil.

**[Table 8]**

| Fatty acids (%) | | WT | N5 | N5-2 | N5-13 | N5-15 | Palm oil |
|---|---|---|---|---|---|---|---|
| C14:0 | Myristic acid | 0.65 | 0.08 | 0.08 | 0.08 | 0.64 | 1.10 |
| C16:0 | Palmitic acid | 35.89 | 40.51 | 41.65 | 41.89 | 39.59 | 44.00 |
| C16:1 | Palmitoleic acid | 3.63 | 3.60 | 4.16 | 4.21 | 3.76 | 0.20 |
| C18:0 | Stearic acid | 5.50 | 3.93 | 2.88 | 3.18 | 3.76 | 4.40 |
| C18:1n9c | Oleic acid | 46.74 | 47.25 | 46.32 | 45.84 | 46.84 | 39.20 |
| C18:2n6c | Linoleic acid | 3.37 | 2.38 | 2.63 | 2.53 | 3.25 | 9.70 |
| Other fatty acids | | 4.22 | 2.24 | 2.28 | 2.27 | 2.17 | 1.40 |

Strain N5-15, which was demonstrated to have the highest oil productivity, was deposited on December 14, 2022, at the National Institute of Technology and Evaluation, Patent Microorganisms Depositary, under accession number NITE BP-03796.

### Industrial Applicability

The present invention is useful in an industrial method for producing a palm oil alternative that can be used in applications such as edible oils and cosmetic ingredients.

## Claims

1. A high oil-producing strain of yeast belonging to the genus *Lipomyces,* the high oil-producing strain being deposited at the National Institute of Technology and Evaluation, Patent Microorganisms Depositary, under accession number NITE BP-03796.

2. A method of producing an oil, the method comprising using a high oil-producing strain of oleaginous yeast belonging to the genus *Lipomyces,* the high oil-producing strain being deposited at the National Institute of Technology and Evaluation, Patent Microorganisms Depositary, under accession number NITE BP-03796.
